# EUROPEAN PATENT SPECIFICATION

(11) **EP 1 653 819 B1**
(45) Date of publication and mention of the grant of the patent: **07.10.2009**
(21) Application number: 04775303.3
(22) Date of filing: 11.08.2004
(51) Int. Cl.: A42B 3/04, G01N 33/44

(54) **UV AGEING INDICATOR DEVICE**
UV ALTERUNGS-ANZEIGEVORRICHTUNG
DISPOSITIF UV D'INDICATION DE DURÉE DE VIE

(30) Priority: 13.08.2003 SE 0302216
(43) Date of publication of application: 10.05.2006
(73) Proprietor: 3M Svenska Aktiebolag, 191 89 Sollentuna (SE)
(72) Inventor: FOLKESSON, Jan, S-331 35 Värnamo (SE)
(74) Representative: Wallengren, Yngvar
(86) International application number: PCT/SE2004/001181
(87) International publication number: WO 2005/016045

(56) References cited:
- WO-A1-95/01896
- PATENT ABSTRACTS OF JAPAN vol. 1998, no. 16 & JP 10 036 690 A (DAITO DENZAI) 10 February 1998

## Description

### TECHNICAL FIELD

The present invention relates to an indicator device for determining the ageing of objects of plastic which are degradable by UV radiation, the indicator device being fixedly disposed on or integrated in the object, and the indicator device includes pigment which degrades by UV radiation and whose degradation follows the degradation, caused by the UV radiation, of the plastic material included in the object, or corresponds to a time interval which is predetermined by legislation or other regulations.

The present invention also relates to a safety helmet including an indicator device.

### BACKGROUND ART

Plastic is a material that is used in many contexts. In particular, it has come into use in the manufacture of different objects for safety and personal protection. Plastic enjoys many advantageous properties, for example that it withstands impact and is readily formed into different shapes on manufacture.

Unfortunately, plastic also has its drawbacks. A not inconsiderable drawback is that it degrades - ages - under the action of ultra violet radiation. For example, the presence of UV radiation may lead to cracks in plastics of different types. Granted, attempts are made to counteract this ageing by material development, but for those materials in existence today ageing is a factor that must be taken into account in the manufacture of parts which are vital to safety, for example personal protection equipment. A few examples of such products are safety helmets, children's car scats and bicycle seats for children, which may all be subjected to UV radiation to a greater or lesser extent throughout their service life.

Degradation in the absence of UV radiation, for example when the products are packed and stored, is however minimal. Differences in the speed of ageing have also been observed depending on whether the product is used indoors or outdoors and whether they are used in northerly or southerly latitudes. In other words, that UV radiation to which the product is exposed is a decisive factor in the speed of ageing.

A date marking which, in certain cases, is employed at present, thus does not reflect the ageing of the product in an adequate manner. In order to be certain that a product that has aged and whose mechanical strength is defective is no longer used, the date marking must be such that considerable safety margins are applied. The limit for how long a product can be used must be set taking as a point of departure the assumption of maximum exposure to UV radiation and thereby consequential ageing. This leads, in many cases, to products that are usable, because they have not been exposed to UV radiation and thereby have not aged at all, becoming unsellable when their date marking has expired. Often, the date marking is respected since no-one wishes to risk injury to a person or damage to property if a product that has become defective because of ageing is used. This entails considerable economic losses for both manufacturers, retailers and end-users who have large quantities of such products in store.

A further problematic aspect is that certain countries have in place extremely stringent legislative requirements for the maximum permitted period of use. This may entail that one and the same product, because of legislation, may on one market at most be used for a short period of time, while on another market it may be used for a longer period of time. Since the actual durability of the product in general exceeds the longer period of time, it is of particular importance to actually utilise the maximum permitted period of time to the full, in order to avoid unnecessary economic losses, at the same as respecting the legislation that is in place.

JP 10-036690 and Patent Abstracts of Japan vol. 1998, No. 16 discloses the use of pigments in association with outdoor articles which are kept permanently outdoors.

### PROBLEM STRUCTURE

Thus, the present invention has for its object to realise an indicator device which better reflects the degree of ageing than prior art date marking.

### SOLUTION

The object foaming the basis of the present invention will be attained if the indicator device intimated by way of introduction is characterised in that the material thickness of the indicator device varies over its surface, for a stepwise degradation of the pigment.

Regarding the safety helmet, the object according to the present invention will be attained if the indicator device displays properties in accordance with at least appended Claim 1 or 2. Further advantages will be attained if the indicator device is moreover given one or more of the characterising features as set forth in appended Claims 3 to 6.

### BRIEF DESCRIPTION OF THE ACCOMPANYING DRAWINGS

The present invention will now be described in greater detail hereinbelow, with reference to the accompanying Drawings. In the accompanying Drawings:
- Fig. 1A: is a cross section of one embodiment of the indicator device according to the present invention;
- Fig. 1B: is a view corresponding to that of Fig. 1A of a variation of the indicator device;
- Fig. 2A: is a plan view of a second embodiment of an indicator device according to the present invention, in its pristine state;
- Fig. 2B: is a view according to Fig. 2A of the indicator device according to the present invention in an aged state;
- Fig. 3: is a straight side elevation of a safety helmet on which an indicator device according to the invention is disposed;
- Fig. 4: is a perspective view of a second embodiment of the indicator device according to the present invention; and
- Fig. 5: is a perspective view of a third embodiment of the indicator device according to the present invention.

### DESCRIPTION OF PREFERRED EMBODIMENT

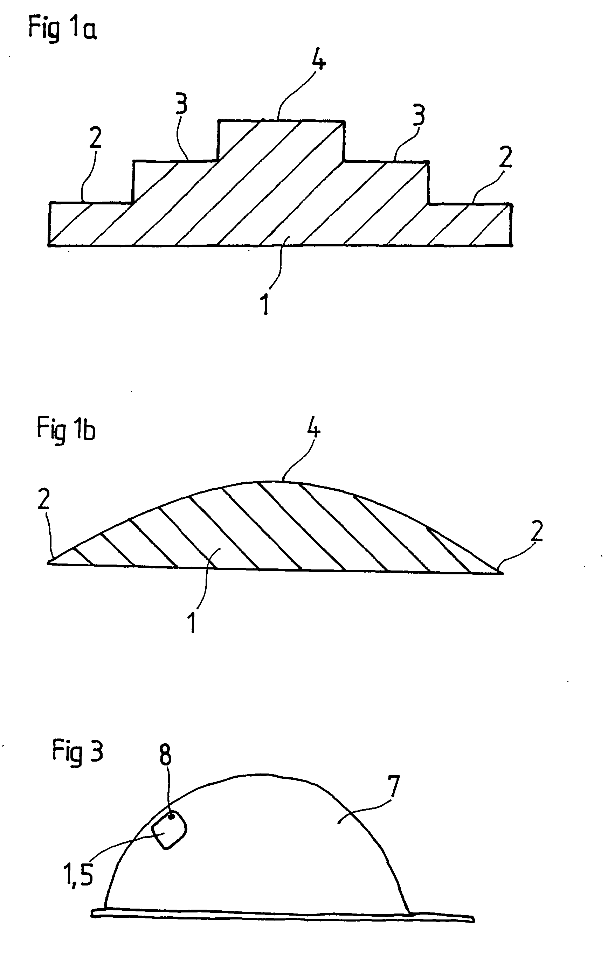

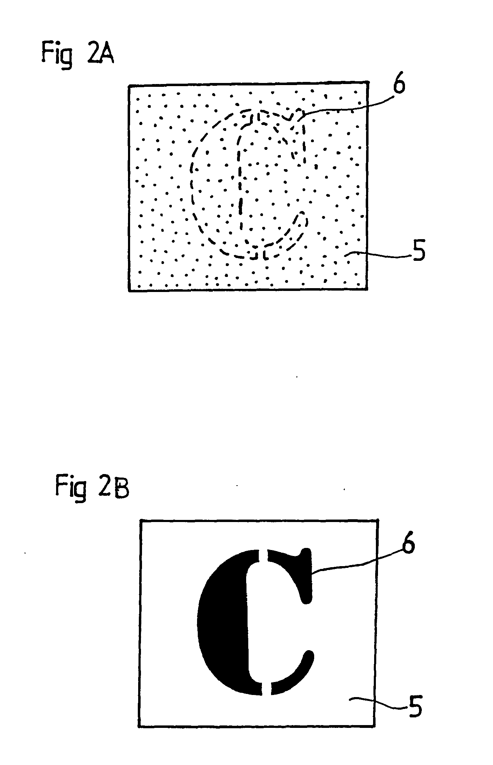

Figs. 1A, 1B, 2A and 2B show examples of embodiments of indicator devices according to the invention. A feature common to these and other embodiments of the present invention is that the indicator device is produced from a carrier material, typically a plastic material, to which is admixed a pigment of a known fastness to light.

Most preferably, the carrier material is polyethylene, since this material is economical, environmentally friendly, is capable of being injection moulded, simple to UV stabilise, simple to colour with the aid of pigment, and also offers a favourable environment for the pigment during the service life of the indicator device. On UV stabilisation, it must be ensured that the pigment is not affected, but that it maintains those properties which are intended.

The pigment carried by the carrier material is such that it degrades more quickly than its carrier material. The speed of degradation under the action of light, in particular ultraviolet light, is indicated by the fastness to light of the pigment. Fastness to light is graded on a scale from 1 to 8 where the lower figures indicate that the pigment degrades rapidly under the action of the light, while the higher figures indicate that the degradation takes longer time. The properties of a number of pigments are well known and documented. For example, it is possible to realise colour shifts from violet to white or red to transparent under the action of a suitable known quantity of UV radiation.

The degradation of the pigment, and thereby the colour of the indicator device, is also affected by the material thickness of the indicator device, since that pigment which lies most proximal the surface of the indicator device is affected first and to the greatest extent, while the pigment lying deeper is affected only when the surface pigment has been broken down. Thus, the colour tends to disappear first in areas of thin material thickness, while areas of greater material thickness maintain the pristine colour longest.

For the indicator device according to the present invention to indicate the degree of ageing under the action of UV radiation on the object, the degradation of the pigment should take place at substantially the same rate as the material in the object ages. Possibly, the degradation of the pigment in the indicator device may be allowed to take place somewhat quicker, but should not take place more slowly, since the intention is to be certain that the material in the object is intact if the indicator device so indicates.

Suitably, the properties of the colour pigment and the material thickness of the indicator device are matched to that degradation which takes place in the material in the object on which the indicator device is disposed. However, the indicator device proper need not be manufactured from the same material as the object; what is vital is that the behaviour of the colour pigment, i.e. degradation under the action of UV radiation, corresponds to that degradation, for example crack formation, which takes place in the manufacturing material of the object itself. In order to cause the degradation of the pigment in the indicator device to correspond to the degradation of the material in the object, the following factors are principally experimented with: choice of pigment, taking into account its properties in relation to light, weather and temperature; the concentration of the pigment, as well as the thickness of the object involved.

Another possibility of adaptation is to select colour pigment and material thickness in such a manner that the degradation of the colour pigment corresponds to a maximum permitted exposure to UV light on a given market. In such instance, the point of departure must be the calculated total UV radiation during the permitted exposure time. Thus, an indicator device may be obtained which indicates when an active use (i.e. exposure to UV radiation) during a predetermined period of time has been achieved. One and the same object may be provided with different indicator devices for an adaptation to the legislation or other regulations applicable on different market. When an object is not used for a period of time, its ageing is discontinued and also the degradation of the colour pigment in the indicator device, provided that it is protected from UV radiation, i.e. is stored indoors.

Fig. 1A shows a cross section of an indicator device 1 of varying material thickness. The material thickness varies gradually from the thinner portions 2, via the medium-thick portions 3 to a portion 4 of maximum thickness. Fig. 1B shows a cross section of a variation of the indicator device 1, where the material thickness varies steplessly between thin edge portions 2 and the central portion 4 of maximum thickness. According as the pigment in the indicator device 1 according to both Figures degrades, its colour will gradually disappear, most rapidly in the thinner portions 2 at the edges and last in the thickest portion 4. The gradual disappearance of the colour may give the user an indication of how long a service life is left for the object on which the indicator device 1 is secured.

Fig. 2A shows a variation of the indicator device whose particular properties may possibly be combined with those properties which have just been described for the indicator device 1 in Fig. 1. The indicator device 5 in Fig. 2A is in the form of a small token which is manufactured from a pigmented carrier material. The pigment is represented in Fig. 2A by a series of dots. In Fig. 2A, the indicator device 5 is shown in its pristine state, i.e. when it and the object on which it is secured are new and have not yet been exposed to substantial quantities of UV radiation. The indicator device 5 is provided with a marking 6 which, in the Figure, is in the form of the letter C. In Fig. 2A, the marking 6 is concealed by the pigment disposed in the indicator device 5. The marking disappears particularly well to the eye if its colour corresponds to the colour of the undegraded pigment. The marking can be cast in the indicator device 5, but may also be retro-applied, for example printed or glued on the underside of the indicator device.

Fig. 2B shows the indicator device 5 according to Fig. 2A after the indicator device 5 has been exposed to UV radiation during a lengthy period of time, in which event its pigment has degraded so that the marking 6 has become visible to the eye. On the manufacture of such an indicator device 5, the marking 6 must be faster to light than the pigment disposed in the indicator device, so that the colour of the marking 6 is not degraded by the UV radiation as quickly as the pigment. When the marking 6 has become readable, this constitutes a signal to the user that the object on which the indicator device 5 is secured is deemed to have been consumed and should be replaced.

Fig. 3 shows an indicator device 1, 5, placed on an object, more precisely a helmet 6 whose ageing should be readable on the indicator device 1, 5. Naturally, it is also possible to place the device 1, 5 on other types of objects.

The indicator device 1, 5 must be well exposed to UV light, preferably exposed to the same degree as those parts of the helmet 7 which are subjected to maximum exposure. By such means, it will be ensured that the material in the helmet 7 has not degraded more than the pigment in the indicator device 1, 5.

The indicator device 1, 5 is secured to the helmet 7 by some form of fixing device 8. Such a fixing device 8 can be realised by riveting, upsetting of material, casting, gluing or other available method. One condition for the method which is employed is that it satisfies other safety requirements that are placed by the authorities and other bodies on the helmet 7. The indicator device 1, 5 should be difficult to remove, it should at least not be possible to replace the indicator device 1, 5 or tamper with it without this being clearly visible. By such means, the risk is avoided that an aged safety helmet 7 is provided with a new indicator device 1, 5 which does not reflect the actual state of the material in the helmet.

### DESCRIPTION OF ALTERNATIVE EMBODIMENTS

One method of varying the indicator device 1, 5 according to the invention is to allow the pigment to be a homogeneous mixture of two or more different pigments with different fastness to light. The result will then be that the indicator device displays different colours according as it is exposed to UV light. For example, it is possible to mix red and yellow pigment where the yellow is faster to light than the red. Initially, the indicator device will appear as red, but as the red pigment disappears, the indicator device changes to yellow. In due course, the yellow coloured pigment will also disappear and the indicator device changes to white. This gives the user a forewarning that the service life of the indicator device 1, 5 and the object 7 on which it is secured is about to be consumed. As a result, new objects can be ordered in good time, which is an advantage on planned purchasing and stock-keeping. The homogeneous mixture of different coloured pigments may advantageously be combined with an indicator device 1, 5 whose thickness varies in accordance with Fig. 1 and/or is provided with an inlaid marking in accordance with Figs. 2A and 2B. Thus, desired visual effects may be obtained with a very large degree of variation.

Yet another method of varying the present invention is to disposed different pigments in different sections or layers of the indicator device 1, 5. For example, the sections may be disposed adjacent one another and when they have assumed the same colour, this signals that the object is to be replaced. A further method is to dispose the different pigments in layers on one another so that the pigment of the first layer disappears first, whereafter the pigment of the second layer begins to degrade. In such instance, this gives an additional method of varying the different colour effects of the indicator device 1, 5. The provision of several layers or sections of different pigments presupposes however a two- or multi-component injection moulding. These variations may also be combined with the variations of the indicator devices 1, 5 where their thickness is varied, a marking 6 is inlaid or where a plurality of different pigments occurs in a homogeneous mixture.

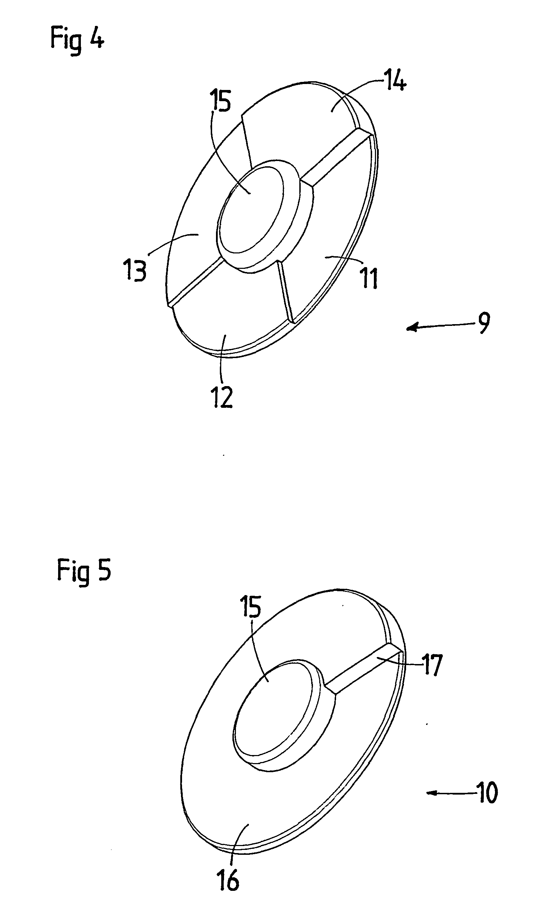

Two concrete exemplifying variations 9 and 10 of the indicator device are shown in Figs. 4 and 5.

The variation which is shown in Fig. 4 has substantially the outer configuration of a round disk or plate. Around a central region 15, there are disposed several sectors 11, 12, 13, 14 of mutually different thicknesses, in this Figure four in number. The pigment will degrade first in the thinnest sector and last in the central region 15 which is thickest. Thus, different sections 11, 12, 13, 14, 15 of the indicator device 9 will assume different colours and the user will thereby be given an indication of how great a part of the total service life or period of use has expired. This indication is further reinforced if the marking 6 which is shown in Figs. 2A and 2B is embedded in the indicator device 9 or is printed on its rear side so that it becomes visible on the degradation of the pigment. For example, a marking can indicate the estimated remaining service life which the object can be actively used before it is consumed, on condition that exposure to UV light is the same.

The variation 10 in Fig. 5 has, apart from a central region 15, a surrounding zone 16 whose thickness increases continuously in a clockwise direction until a complete circle has been passed and a discontinuity in the form of a step 17 occurs. The colour change on the degradation of the pigment will take place gradually in the same direction as the thickness of the zone 16 increases. In the same manner as described above, markings 6 may be embedded in or printed on the indicator device 10 in order to give the user an indication of when the object should be replaced.

While the indicator device 1, 5 in Fig. 3 has been shown as secured on a safety helmet 7, it is naturally possible to dispose the indicator device 1, 5, 9, 10 on other objects where it is important to monitor the ageing of the material. This applies in particular to objects where the safety aspect is crucial, for example bicycle pillion seats for children and children's safety seats in cars, as was mentioned by way of introduction.

The present invention may be varied further without departing from the scope of the appended Claims.

## Claims

1. An indicator device for determining the ageing of an object (7) of plastic which is degradable by UV radiation, the indicator device (1, 5, 9, 10) being fixedly disposed on or integrated in the object (7), the indicator device (1, 5, 9, 10) including pigment which degrades by UV radiation and whose degradation follows the degradation of the plastic material included in the object (7) caused by UV radiation, **characterised in that** the material thickness (2, 3, 4, 11, 12, 13, 14, 15, 16) of the indicator device varies over its surface, for a stepwise degradation of the pigment.

2. An indicator device for determining the ageing of an object (7) of plastic which is degradable by UV radiation, the indicator device (1, 5, 9, 10) being fixedly disposed on or integrated in the object (7), the indicator device (1, 5, 9, 10) including pigment which degrades by UV radiation and whose degradation corresponds to a time interval which is predetermined by legislation or other regulations, **characterised in that** the material thickness (2, 3, 4, 11, 12, 13, 14, 15, 16) of the indicator device varies over its surface, for a stepwise degradation of the pigment.

3. The indicator device (1, 5, 9, 10) as claimed in Claim 1 or 2, **characterised in that** the indicator device (1, 5, 9, 10) includes a marking (6) which is concealed by the pigment and which becomes visible on degradation thereof

4. The indicator device (1, 5, 9, 10) as claimed in any of Claims 1 to 3, **characterised in that** the pigment is a mixture of several components of different degradation properties for realising a multi-stage colour indication.

5. The indicator device (1, 5, 9, 10) as claimed in any of Claims 1 to 4, **characterised in that** the device includes a plurality of layers in which the pigments or component mixtures differ for a stepwise degradation of the pigments.

6. A safety helmet (7) including an indicator device (1, 5, 9, 10), **characterised in that** the indicator device (1, 5, 9, 10) displays properties as claimed in any of Claims 1 to 5.

## Patentansprüche

1. Anzeigevorrichtung zur Bestimmung des Alterns eines Objektes (7) aus Kunststoff, welches durch UV Strahlung degradierbar ist, wobei die Anzeigevorrichtung (1, 5, 9, 10), fest angeordnet ist auf oder integriert ist in das Objekt (7) und die Anzeigevorrichtung (1, 5, 9, 10) Pigment umfasst, welches bei UV Strahlung degradiert und dessen Degradierung der von UV Strahlung verursachten Degradierung des Kunststoffmaterials folgt, welches im Objekt (7) enthalten ist, **dadurch gekennzeichnet, dass** die Materialdicke (2, 3, 4, 11, 12, 13, 14, 15, 16) der Anzeigevorrichtung über ihre Fläche für eine schrittweise Degradierung des Pigments variiert.

2. Anzeigevorrichtung zur Bestimmung der Alterung eines Objektes (7) aus Kunststoff, welches durch UV Strahlung degradierbar ist, wobei die Anzeigevorrichtung (1, 5, 9, 10), fest angeordnet ist auf oder integriert ist in das Objekt (7) und die Anzeigevorrichtung (1, 5, 9, 10) Pigment umfasst, welches bei UV Strahlung degradiert und dessen Degradierung einem Zeitintervall entspricht, welches durch Gesetzgebung oder anderen Regulierungen vorbestimmt ist, **dadurch gekennzeichnet, dass** die Materialdicke (2, 3, 4, 11, 12, 13, 14, 15, 16) der Anzeigevorrichtung über ihre Fläche für eine schrittweise Degradierung des Pigments variiert.

3. Anzeigevorrichtung (1, 5, 9, 10) nach Anspruch 1 oder 2, **dadurch gekennzeichnet, dass** die Anzeigevorrichtung (1, 5, 9, 10) eine Markierung (6) umfasst, welche durch das Pigment verdeckt ist und welche durch Degradierung sichtbar wird.

4. Anzeigevorrichtung (1, 5, 9, 10) nach einem der Ansprüche 1 bis 3, **dadurch gekennzeichnet, dass** das Pigment eine Mischung aus mehreren Komponenten mit unterschiedlichen Degradierungseigenschaften zur Realisierung einer vielstufigen Farbindikation ist.

5. Anzeigevorrichtung (1, 5, 9, 10) nach einem der Ansprüche 1 bis 4, **dadurch gekennzeichnet, dass** die Vorrichtung eine Mehrzahl von Schichten umfasst, in welchen die Pigmente oder Komponentenmischungen für eine schrittweise Degradierung der Pigmente differieren.

6. Sicherheitshelm (7) umfassend eine Anzeigevorrichtung (1, 5, 9, 10), **dadurch gekennzeichnet, dass** die Anzeigevorrichtung (1, 5, 9, 10) Eigenschaften nach einem der Ansprüche 1 bis 5 anzeigt.

## Revendications

1. Dispositif formant indicateur destiné à déterminer le vieillissement d'un objet (7) en matière plastique qui se dégrade sous l'effet d'un rayonnement UV, le dispositif formant indicateur (1, 5, 9, 10) étant disposé à demeure sur l'objet (7) ou intégré dans ce dernier, le dispositif formant indicateur (1, 5, 9, 10) comportant un pigment qui se dégrade sous l'effet d'un rayonnement UV et dont la dégradation suit la dégradation de la matière plastique contenue dans l'objet (7), provoquée par le rayonnement UV, **caractérisé en ce que** l'épaisseur de matériau (2, 3, 4, 11, 12, 13, 14, 15, 16) du dispositif formant indicateur varie sur sa surface, afin de mettre en évidence une dégradation par palier du pigment.

2. Dispositif formant indicateur destiné à déterminer le vieillissement d'un objet (7) en matière plastique qui se dégrade sous l'effet d'un rayonnement UV, le dispositif formant indicateur (1, 5, 9, 10) étant disposé à demeure sur l'objet (7) ou intégré dans ce dernier, le dispositif formant indicateur (1, 5, 9, 10) comportant un pigment qui se dégrade sous l'effet d'un rayonnement UV et dont la dégradation correspond à un intervalle de temps qui est prédéterminé par la législation ou autres réglementations, **caractérisé en ce que** l'épaisseur de matériau (2, 3, 4, 11, 12, 13, 14, 15, 16) du dispositif formant indicateur varie sur sa surface, afin de mettre en évidence une dégradation par palier du pigment.

3. Dispositif formant indicateur (1, 5, 9, 10) selon la revendication 1 ou 2, **caractérisé en ce que** le dispositif formant indicateur (1, 5, 9, 10) comporte un repère (6) qui est masqué par le pigment et qui devient visible lors de la dégradation de ce dernier.

4. Dispositif formant indicateur (1, 5, 9, 10) selon l'une quelconque des revendications 1 à 3, **caractérisé en ce que** le pigment est un mélange de plusieurs composants de propriétés de dégradation différentes afin de réaliser une indication de couleur à plusieurs paliers.

5. Dispositif formant indicateur (1, 5, 9, 10) selon l'une quelconque des revendications 1 à 4, **caractérisé en ce que** le dispositif comporte une pluralité de couches dans lesquelles les pigments ou mélanges de composants diffèrent afin de mettre en évidence une dégradation par palier des pigments.

6. Casque de sécurité (7) comprenant un dispositif formant indicateur (1, 5, 9, 10), **caractérisé en ce que** le dispositif formant indicateur (1, 5, 9, 10) présente des propriétés selon l'une quelconque des revendications 1 à 5.
